(19) **Europäisches Patentamt / European Patent Office / Office européen des brevets**

(11) **EP 4 613 374 A1**

(12) **EUROPEAN PATENT APPLICATION**
published in accordance with Art. 153(4) EPC

(43) Date of publication:
10.09.2025 Bulletin 2025/37

(21) Application number: 23899949.4

(22) Date of filing: 05.12.2023

(51) International Patent Classification (IPC):
*B01J 27/051* $^{(2006.01)}$   *B01J 27/049* $^{(2006.01)}$
*B01J 27/047* $^{(2006.01)}$   *C10G 45/08* $^{(2006.01)}$

(52) Cooperative Patent Classification (CPC):
B01D 53/46; B01D 53/62; B01D 53/86;
B01J 27/047; B01J 27/049; B01J 27/051;
C10G 2/00; C10G 45/08; Y02P 20/52

(86) International application number:
PCT/CN2023/136371

(87) International publication number:
WO 2024/120373 (13.06.2024 Gazette 2024/24)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 08.12.2022 CN 202211569861

(71) Applicants:
• China Petroleum & Chemical Corporation
Beijing 100728 (CN)
• Sinopec Dalian Research Institute of
Petroleum and Petrochemicals Co., Ltd.
Lushunkou District
Dalian, Liaoning 116045 (CN)

(72) Inventors:
• SUN, Jin
Dalian, Liaoning 116045 (CN)
• GUO, Rong
Dalian, Liaoning 116045 (CN)
• LI, Yang
Dalian, Liaoning 116045 (CN)
• NIU, Shikun
Dalian, Liaoning 116045 (CN)
• DUAN, Weiyu
Dalian, Liaoning 116045 (CN)

(74) Representative: karo IP
Patentanwälte PartG mbB
Steinstraße 16-18
40212 Düsseldorf (DE)

(54) **HYDROGENATION CATALYST AND PREPARATION METHOD THEREFOR AND USE THEREOF**

(57) The inwention discloses a dry gas hydrogenation catalyst and a preparation method therefor. The dry gas hydrogenation catalyst comprises a carrier component and an active hydrogenation component; the carrier component comprises aluminum oxide; and the active hydrogenation component is present in the form of a sulfide on the catalyst, the average number of lamella layers of the sulfide is less than 1.5, and the proportion of a single layer is 65%-80%. The preparation method comprises the following steps: (I) taking aluminum oxide powder A and roasting same at high temperature in an oxygen-containing atmosphere to obtain aluminum oxide dry gel powder B; II) impregnating the aluminum oxide dry gel powder B using the active hydrogenation component impregnating solution C, drying, roasting at high temperature in the oxygen-containing atmosphere, and sulfidizing same to obtain powder E; and III) molding, drying, and roasting the powder E prepared in step (II), wherein the respective temperature of high-temperature roasting in step I) and step II) are 800°C or above. The catalyst prepared by the method has good performance in removing impurities such as oxygen, CO, and $CO_2$, and also has good organic sulfur and olefin hydrogenation performance.

EP 4 613 374 A1

FIG. 1

**Description**

**CROSS-REFERENCE TO RELATED APPLICATIONS**

The application claims the benefit of the Chinese patent application No. "202211569861.2", filed on December 8, 2022, the contents of which are specifically and entirely incorporated herein by reference.

**TECHNICAL FIELD**

The present invention relates to the hydrogenation field, in particular relates to a hydrogenation catalyst and a preparation method therefor, and use thereof.

**BACKGROUND ART**

There are many refinery enterprises in China, which have abundant light hydrocarbon sources such as catalytic dry gas and coking dry gas. For example, the coking dry gas has a high content of impurities such as oxygen, sulfur, CO, and $CO_2$, thus the coking dry gas lacks a more profitable usage other than the use as fuel and the use for hydrogen production. When the coking dry gas is subjected to hydroprocessing to perform hydrogenation saturation on the diolefin and olefin contained therein and remove the impurities (e.g., sulfur, oxygen), the processed gas can be used as a high quality feedstock for olefin production, it has a high yield of triene, such a process can not only compensate the shortage of ethylene feedstock , but also improve the economic efficiency.

CN101722006A with an application number "200910230393.4" discloses a dual-functional hydrogenation catalyst applied in a hydrogenation refining process for high-olefin raw materials such as coking dry gas or catalytic dry gas has good organic sulfur hydrogenolysis and olefin saturation performance. The catalyst uses Co-Mo-Ni-Cu-rare earth active metal loaded by titanium oxide-aluminum oxide , which can efficientively hydrogenate organic sulfur and olefin of the coking dry gas and catalytic dry gas.

CN112742399A with an application number "201911052623.2" discloses a dry gas hydrodesulfurization catalyst as well as a preparation method and application thereof. The catalyst comprises a raspberry-type particles composed of an active component a, a structural auxiliary agent b, and other auxiliary agents c, the raspberry-type particle is hollow microspheres with a macropore on its surfaces, the hollow microsphere is internally provided with hollow structures, and the macropore is connected with the hollow structure to form a cavity with one ends opening; the metal element of the active component a is selected from Ni and Mo; the structural auxiliary agent b is selected from one or more of aluminum oxide, silicon oxide, titanium oxide, and zirconium oxide; and the metal element of the other auxiliary agent c is selected from one or more of Cu, La, Ce, W, Mn, and Zn.

CN112742408A with an application number "201911053316.6" discloses a dry gas hydrogenation saturated olefin and desulfurization catalyst as well as a preparation method and application thereof. The catalyst comprises a carrier and an active metal component loaded on the carrier, the active metal component comprises molybdenum and a VIII group metal, based on the catalyst, the content of molybdenum is 10-45 wt% in terms of oxide, the content of the VIII group metal is 1-10 wt% in terms of oxide, and the content of the carrier oxide is 50-89 wt%, preferably 55-80 wt%.

Extensive researches and experiments have demonstrated that the content of oxygen, CO, $CO_2$, and other impurities in the dry gas will affect the hydrogenation activities of the organic sulfur and olefin of the catalyst, thus the catalyst must have desirable impurity-removal performance when the content of oxygen, CO, $CO_2$, and other impurities in the dry gas is high.

**SUMMARY OF THE INVENTION**

To overcome the defects in the prior art, the present invention proposes a hydrogenation catalyst and a preparation method therefor, the catalyst has desirable hydrogenation performance, in particular for hydrogenation of organic sulfur and olefin, for example, particularly suitable for application in hydrogenation refining processes of low carbon hydrocarbons, such as hydrogenation of ethylene cracking raffinate, liquefied gas, or dry gas, and in preferred embodiments, the catalyst has good performance in removing impurities such as oxygen, CO, and $CO_2$, and also has desirable organic sulfur and olefin hydrogenation performance by introducing a coagent, according to the different components that need to be removed in the reaction feedstocks.

The present invention provides a hydrogenation catalyst comprising a carrier component and an active hydrogenation component; the carrier component comprises aluminum oxide; and the active hydrogenation component is present in the form of a sulfide on the catalyst, the average number of lamellar layers of the sulfide is less than 1.5, and 1.2-1.3 layers are preferred, and the proportion of a single layer is within the range of 65%-80%, such as 65%, 70%, 72%, 75%, 78%, 80%. The catalyst having the aforementioned structural features of the invention exhibits an excellent carbon deposit resistance advantage. Its application to the hydrogenation process can produce the effects of good stability and long service life of process operation.

To ensure that the catalyst has good performance in removing impurities such as oxygen, CO, and $CO_2$, it is preferred that the hydrogenation catalyst comprises a co-agent, which can be selected from a wide range, the co-agent is exemplified below, but it is not intended to limit the advantages of the invention, the co-agent comprises one or more of Cu, Ag, Ni, Co, W, and Mo. The advantages of the invention are exemplified herein by one or both of Cu, Ag, Ni, Co, and W, but do not impose limitations to the protection scopes of the invention.

According to a preferred embodiment of the invention, preferably, the coagent is present in the form of a metallic state on the catalyst. The presence of the co-agent in the form of a metallic state enables hydrogenation and removal of impurities (e.g., oxygen, CO, $CO_2$) at a lower temperature.

In the invention, the content of said co-agent can be selected from a wide range, and the conventional content range may be used to achieve the objects of the invention, the content is exemplified below, but it is not intended to limit the ranges of the invention; according to a preferred embodiment of the invention, the coagent content in the catalyst is preferably from 0.1wt% to 4wt%, more preferably from 0.3wt% to 4wt%, further preferably from 0.5wt% to 3wt%, such as 0.5wt%, 0.8wt%, 1wt%, 1.4wt%, 1.6wt%, 1.7wt%, 1.8wt%, 2.0wt%, 2.5wt%, 2.8wt%, or 3wt%.

In the invention, the content of the active hydrogenation component can be selected from a wide range, and the conventional content range may be used to achieve the objects of the invention, the content is exemplified below, but it is not intended to limit the ranges of the invention; according to a preferred embodiment of the invention, the content of active hydrogenation component calculated in terms of sulfide in the total mass of the catalyst is preferably from 1.5wt% to 30wt%, more preferably from 10wt% to 20wt%, such as 10wt%, 11wt%, 12wt%, 14wt%, 16wt%, 17wt%, 18wt%, 20wt%, based on the weight of said catalyst.

The invention does not impose particular requirements on the selections of the active hydrogenation component, the common types of active hydrogenation components are applicable to the present invention, and the types of active hydrogenation components are exemplified below, but are not intended to limit the ranges of the invention; according to a preferred embodiment of the invention, the active hydrogenation component comprises Group VIB metals and Group VIII metals; preferably, GroupVIB metals comprise Mo and/or W; Group VIII metals comprise Co and/or Ni. Mo and Co, Mo and Ni, and W, Mo and Ni are exemplified in examples of the invention to illustrate the advantages of the present invention but do not limit the scopes of the present invention.

The invention does not impose particular requirements on the dosage of the active hydrogenation component, the common dosage ranges are applicable to the present invention, the common dosage ranges are exemplified below, but are not intended to limit the ranges of the invention; according to a preferred embodiment of the invention, the content of Group VIB metal sulfide in the total mass of the catalyst is preferably from 1wt% to 20wt%, more preferably from 8wt% to 16wt%, the content of Group VIII metal sulfide in the total mass of the catalyst is preferably from 0.5wt% to 10wt%, more preferably from 2wt% to 4wt%, based on the weight of the catalyst.

According to a preferred embodiment of the invention, the total acid amount of the catalyst is within the range of 0.1-0.4 mmol/g, wherein the content of strong acid amount at 400-500°C is within the range of 5-15%, the content of moderate strength acid amount at 250-400°C is within the range of 10-20%, and the balance is weak acid at 150-250°C. The catalyst having the aforementioned acid content characteristic exhibits the advantage of reducing the acid cleavage side reaction thereby decreasing the carbon deposit of the catalyst. The application of said catalyst to the hydrogenation process can produce the desired effect of increasing the operating life of the catalyst.

The invention has no particular requirement on the preparation method of a hydrogenation catalyst, any of the catalysts satisfying the aforementioned characteristic requirements can achieve the object of the invention, the invention provides a preparation method of a hydrogenation catalyst, the preparation method comprises the following steps:

(I) taking aluminum oxide powder A and roasting same at high temperature in an oxygen-containing atmosphere to obtain aluminum oxide dry gel powder B;
(II) impregnating the aluminum oxide dry gel powder B by using the active hydrogenation component impregnating solution C, drying, roasting at high temperature in the oxygen-containing atmosphere, and sulfidizing same to obtain powder E;
(III) molding, drying, and roasting the powder E prepared in step (II);

wherein the respective temperature of high-temperature roasting in step (I) and step (II) are 800°C or above. Step (I) of the invention initially relates to roasting the aluminum oxide powder to promote lattice deformation of the aluminum oxide; an active hydrogenation component in step (II) after the roasting process will not migrate or aggregate during the vulcanization process, thereby obtaining the effect of the invention that an average number of lamella layers of the sulfide is less than 1.5.

In the present invention, it is preferable that the method further comprises the following steps: impregnating the aluminum oxide powder A with a coagent impregnating solution D, drying the impregnated aluminum oxide powder A and reducing to obtain powder F; step (III) comprises: mixing the powder E with the powder F, and then molding, drying, and roasting the mixture.

The invention does not impose specific requirements on the drying and reducing steps, the steps are exemplified below but are not intended to limit the ranges of the invention.

According to a preferred embodiment of the invention, the reduction treatment includes roasting in a reducing atmosphere, more preferably roasting in a hydrogen atmosphere.

According to a preferred embodiment of the invention, the roasting conditions in the reduction treatment comprise a roasting temperature within the range of 300-700°C and a roasting time within the range of 2-5 hours.

According to a preferred embodiment of the present invention, the drying conditions before reduction comprise a drying time within the range of 1-5 hours and a drying temperature within the range of 80-120°C.

The invention does not impose specific requirements on the impregnation method, the contents are exemplified below, but are not intended to limit the ranges of the invention; preferably, the impregnation method of the coagent impregnation is saturated impregnation or supersaturated impregnation.

In the present invention, the aluminum oxide powder A needs to be an active aluminum oxide, physicochemical parameters of the aluminum oxide powder A in the invention preferably comprise: a specific surface area within the range of 100-480 $m^2$/g, a pore volume within the range of 0.4-1.2 $cm^3$/g, and a pore diameter within the range of 4.0-35.0nm, and the dry basis content is, for example, within the range of 60-80wt%; it is exemplified that the aluminum oxide powder A is, for example, a pseudo-boehmite dry gel powder. The pseudo-boehmite dry gel powder A is a commercially available commodity, which can be prepared through an aluminum alcohol method, a sodium metaaluminate neutralization method, a carbonization method, and the like.

The invention does not impose specific requirements on the oxygen-containing atmosphere in step (I), the contents are exemplified below, but are not intended to limit the ranges of the invention; according to a preferred embodiment of the invention, the oxygen-containing atmosphere has an oxygen content from 10 v% to 30 v%, more preferably an air atmosphere.

The object of the present invention can be achieved by selecting the roasting conditions at high temperature in step (I) comprises a roasting temperature of 800°C or higher; the specific roasting temperature is selected and determined according to the practical requirements; regarding the invention, the roasting conditions at high temperature preferably comprise a roasting temperature within the range of 800-1,100°C, for example, 800°C, 850°C, 900°C, 950°C, 1,000°C, 1,050°C, 1,100°C, the roasting time is selected and determined according to the practical requirements; the roasting time is within the range of 0.5-5 hours, for example, 0.5h, 1h, 1.5h, 2h, 2.5h, 3h, 3.5h, 4h, 4.5h, 5h.

The invention does not impose specific requirements on the oxygen-containing atmosphere in step (II), the contents are exemplified below, but are not intended to limit the ranges of the invention; according to a preferred embodiment of the invention, the oxygen-containing atmosphere in step (II) has an oxygen content from 10 v% to 30 v%, more preferably an air atmosphere.

The object of the present invention can be achieved by selecting the roasting conditions at high temperature in step (II) comprising a roasting temperature of 800°C or higher; the specific roasting temperature is selected and determined according to the practical requirements; regarding the invention, the roasting conditions at high temperature preferably comprise a roasting temperature within the range of 800-1,100°C, for example, 800°C, 850°C, 900°C, 950°C, 1,000°C, 1,050°C, 1,100°C, the roasting time is selected and determined according to the practical requirements; the roasting time is within the range of 0.5-5 hours, for example, 0.5h, 1h, 1.5h, 2h, 2.5h, 3h, 3.5h, 4h, 4.5h, 5h.

The invention does not impose specific requirements on the sulfidizing process, the main purpose of said process is vulcanization, the contents are exemplified below, but are not intended to limit the ranges of the invention; according to a preferred embodiment of the invention, the sulfidizing in step (II) comprises: roasting in a vulcanization atmosphere, preferably roasting in a mixed atmosphere of $H_2S$ and $H_2$, and the content of $H_2S$ in the mixed atmosphere of $H_2S$ and $H_2$ is preferably within the range of 0.1 v%-2 v%. The examples of the invention use the content 1 v% as an exemplary illustration, but the scope of the invention is not thus limited.

In the invention, the roasting vulcanization conditions are not specifically limited, the common vulcanization roasting temperature can be suitable for the invention, regarding the invention, preferably, the roasting conditions in the vulcanization atmosphere comprise: a roasting temperature within the range of 250-550°C; the roasting time is determined according to the practical requirements, for example, the roasting time is within the range of 3-8 hours.

In the invention, the impregnation method is not particularly limited, the contents are exemplified below, but are not intended to limit the ranges of the invention; the impregnation method of step (II) is preferably saturated impregnation or supersaturated impregnation.

In the invention, the conditions of molding, drying, and roasting in step (III) can be performed by referring to the prior art, the contents are exemplified below but are not intended to limit the ranges of the invention.

Specifically, for example, the catalyst molding process in step (III) comprises: adding an extrusion aid, a peptizing agent, and water into the composite powder and blending to form a plastic body, then kneading and molding the plastic body.

The type of extrusion aid is not particularly required in the invention, for example, the extrusion aid is one or more selected from the group consisting of methylcellulose, sesbania powder, starch, and polyvinyl alcohol.

The type of peptizing agent is not particularly limited in the invention, for example, the peptizing agent is one or more selected from the group consisting of dilute nitric acid, dilute phosphoric acid, and silicic acid.

The drying conditions are not particularly limited in the invention, for example, the drying conditions in steps (II) and (III) respectively comprise a drying time within the range of 1-5 hours and a drying temperature within the range of 80-120°C.

The roasting conditions are not particularly limited in the invention, the roasting condition in step (III) comprises a roasting temperature within the range of 200-350°C, and the roasting time is determined according to the practical requirements, for example, the roasting time within the range of 1-4 hours.

In the invention, the roasting atmosphere in step (III) is, for example, an inert atmosphere, which is one or more gases selected from $N_2$, He, or Ar.

In the method of the invention, both the active component impregnating solution and the coagent impregnating solution are aqueous solutions prepared with a conventional method. For example, the active component impregnating solution may be selected from the group consisting of aqueous solutions prepared with ammonium molybdate, ammonium metatungstate, cobalt nitrate, nickel nitrate, basic cobalt carbonate, basic nickel carbonate, and other salt; the co-agent impregnating solution may be selected from the group consisting of aqueous solutions prepared with copper nitrate, silver nitrate, ammonium molybdate, ammonium metatungstate, cobalt nitrate, nickel nitrate, basic cobalt carbonate, basic nickel carbonate, and other salts. The selected metal salts are water-soluble, but not limited to the above-mentioned salts. The feeding amount of each component in the impregnating solution is calculated according to the content of each component in the catalyst.

The present inventors have discovered in the research that the metal sulfide with a lower number of layers has desirable hydrodesulfurization activity. When the interaction force between the aluminum oxide as a carrier and the hydrogenation active metal is small, the metal sulfide generated after the vulcanization process has a multilayer structure, and the sulfide with a multilayer structure is migrated and aggregated in the hydrogenation process due to the heat release. The acidity of the aluminum oxide powder after high-temperature treatment is obviously reduced, when the aluminum oxide powder is impregnated in hydrogenation active metal and subjected to roasting at high temperature, the interaction force between the aluminum oxide and the metal is strengthened, the number of stacked layers of the metal sulfide generated after the vulcanization process is obviously reduced, the number of a single layer is significantly increased, and the stability of hydrogenation activity of the catalys is improved.

The hydrogenation catalyst in the invention is suitable for use in various hydrogenation scenarios such as hydrogen of ethylene cracking raffinate oil, liquefied gas, or dry gas, preferably the use in the reactions for producing low carbon hydrocarbons from dry gas hydrogenation.

In the invention, the reaction conditions of the reaction for producing low carbon hydrocarbons from dry gas hydrogenation are not particularly limited, the common conditions are applicable to the invention, preferably, the reaction conditions for the reaction for producing low carbon hydrocarbon from dry gas hydrogenation comprise: a reaction pressure within the range of 0.1-10MPa, a gas hourly space velocity within the range of 300-10,000 $h^{-1}$, and a reaction temperature within the range of 150-400°C.

According to a preferred embodiment of the invention, the dry gas hydration catalyst of the invention comprises a carrier component, an active component, and a co-agent; the carrier component is aluminum oxide; the active components comprise Group VIB metals and Group VIII metals, wherein the Group VIB metals are preferably Mo and/or W; the Group VIII metals are preferably Co and/or Ni; the coagent is one or more of Cu, Ag, Ni, Co, W, and Mo; the active component is present in the form of a sulfide on the catalyst, the average number of lamella layers of the sulfide is less than 1.5, and the proportion of a single layer is within the range of 65%-80%; the coagent is present in the form of a metallic state on the catalyst.

According to a preferred embodiment of the invention, the content of Group VIB group metal sulfide in the total mass of the catalyst is from 1wt% to 20wt%, the content of Group VIII metal sulfide in the total mass of the catalyst is from 0.5wt% to 10wt%, based on the weight of the catalyst.

According to a preferred embodiment of the invention, the coagent content in the catalyst is from 0.1wt% to 4wt%, more preferably from 0.3wt% to 4wt%.

According to a preferred embodiment of the invention, the total acid amount of the catalyst is within the range of 0.1-0.4 mmol/g, wherein the content of strong acid amount at 400-500°C is within the range of 5-15%, the content of moderate strength acid amount at 250-400°C is within the range of 10-20%, and the content of weak acid at 150-250°C is within the range of 65-85%.

According to a preferred embodiment of the invention, a preparation method of dry gas hydrogenation catalyst of the invention comprises the following steps:

(1) taking pseudo-boehmite dry gel powder A, and roasting same at high temperature in an oxygen-containing atmosphere to obtain aluminum oxide dry gel powder B;

(2) impregnating the aluminum oxide dry gel powder B using the active hydrogenation component impregnating solution C, drying, roasting at high temperature in the oxygen-containing atmosphere, then roasting in a mixed atmosphere of $H_2S$ and $H_2$, to obtain powder E;

(3) impregnating the pseudo-boehmite dry gel powder A with a coagent impregnating solution D, and drying the same, then roasting in a hydrogen gas atmosphere to obtain powder F;

(4) uniformly mixing the powder E prepared in step (2) and the powder F prepared in step (3), then molding, drying, and roasting the mixture to prepare a dry gas hydrogenation catalyst.

In the method of the invention, the pseudo-boehmite dry gel powder A in step (1) is a commercially available commodity, which can be prepared through an aluminum alcohol method, a sodium metaaluminate neutralization method, a carbonization method, and the like.

In the method of the invention, the oxygen content in an oxygen-containing atmosphere in step (1) is from 10 v% to 30 v%, preferably an air atmosphere is adopted; the roasting conditions at a high temperature comprise a roasting temperature within the range of 800-1,100°C and a roasting time within the range of 0.5-5 hours.

In the method of the invention, the oxygen content in an oxygen-containing atmosphere in step (2) is from 10 v% to 30 v%, preferably an air atmosphere is adopted; the roasting conditions at a high temperature comprise a roasting temperature within the range of 800-1,100°C and a roasting time within the range of 0.5-5 hours.

In the method of the invention, the roasting condition of the mixed atmosphere of $H_2S$ and $H_2$ in step (2) comprises a roasting temperature within the range of 250-550°C and a roasting time within the range of 3-8 hours; the content of $H_2S$ in the mixed atmosphere of $H_2S$ and $H_2$ is within the range of 0.1 v%-2 v%.

In the method of the invention, the roasting condition in the hydrogen atmosphere in step (3) comprises a roasting temperature within the range of 300-700°C and a roasting time within the range of 2-5 hours.

In the method of the invention, both the active component impregnating solution C in step (2) and the coagent impregnating solution in step (3) are aqueous solutions prepared with a conventional method. For example, the active component impregnating solution C may be selected from the group consisting of aqueous solutions prepared with ammonium molybdate, ammonium metatungstate, cobalt nitrate, nickel nitrate, basic cobalt carbonate, basic nickel carbonate, and other salt; the coagent impregnating solution D may be selected from the group consisting of aqueous solutions prepared with copper nitrate, silver nitrate, ammonium molybdate, ammonium metatungstate, cobalt nitrate, nickel nitrate, basic cobalt carbonate, basic nickel carbonate, and other salts. The selected metal salts are water-soluble, but not limited to the above-mentioned salts. The feeding amount of each component in the impregnating solution is calculated according to the content of each component in the catalyst.

In the method of the invention, the impregnation method in steps (2) and (3) is saturated impregnation or supersaturated impregnation, the method is well-known among those skilled in the art; the drying process is also well-known among those skilled in the art, such as a conventional drying or vacuum drying process.

In the method of the invention, the catalyst molding process in step (4) is a well-known method in the field, for example, adding an extrusion aid, a peptizing agent, and water into the composite powder and blending to form a plastic body, then kneading and molding the plastic body; wherein the extrusion aid is one or more selected from the group consisting of methylcellulose, sesbania powder, starch, and polyvinyl alcohol; the peptizing agent is one or more selected from the group consisting of dilute nitric acid, dilute phosphoric acid, and silicic acid.

In the method of the invention, the drying conditions in steps (2), (3), and (4) comprise a drying time within the range of 1-5 hours and a drying temperature within the range of 80-120°C.

In the method of the invention, the roasting conditions in step (4) comprise a roasting temperature within the range of 200-350°C and a roasting time within the range of 1-4 hours. The roasting atmosphere is an inert atmosphere, which is one or more gases selected from $N_2$, He, or Ar.

The dry gas hydrogenation catalyst is applied in the reaction to produce low carbon hydrocarbons from dry gas hydrogenation, the reaction conditions comprise: a reaction pressure within the range of 0.1-10MPa, a gas hourly space velocity within the range of 300-10,000 $h^{-1}$, and a reaction temperature within the range of 150-400°C. The specific process conditions can be adjusted on the operational conditions according to the quality difference of the raw materials.

The present inventors have discovered in the research that the majority of organic sulfur in the dry gas is carbonyl sulfide, the metal sulfide with a lower number of layers has desirable hydrodesulfurization activity. When the interaction force between the aluminum oxide as a carrier and the Group VIB and/or the Group VIII active metals is small, the metal sulfide generated after the vulcanization process has a multilayer structure, and the sulfide with a multilayer structure is migrated and aggregated in the dry gas hydrogenation process because that the olefin will rapidly saturate and release heat sharply. In addition, the catalyst surface has a strong acidity and contains impurities such as oxygen, CO, and $CO_2$, which can cause olefin polymerization and carbon deposition. The above factors cause a decreased desulfurization activity of the catalyst and olefin saturation activity. The acidity of the aluminum oxide dry gel powder after high-temperature treatment is obviously reduced, when the aluminum oxide dry gel powder is impregnated in the Group VIB and/or the Group VIII hydrogenation active metals and then subjected to roasting at high temperature, the interaction force between the aluminum oxide and the metal is strengthened, the number of stacked layers of the metal sulfide generated after the vulcanization process is obviously reduced, the number of a single layers is significantly increased, and the stability of the catalysis desulfurization and olefin saturation activity is improved.

In addition, if the dry gas contains excessive impurities such as oxygen, CO, and $CO_2$, particularly CO, the impurities will be adsorbed on the active metals to degrade the hydrogenation and desulfurization performance of the catalyst. The present inventors adopt the coagent metal, which has the advantage of adsorbing oxygen, CO, $CO_2$, and other impurities to perform a hydrogenation reaction for the removal of said impurities; in addition, compared with the aluminum oxide powder treated by the roasting at high temperature, the pseudo-boehmite powder not treated at high temperature has better peptization property and can be directly molded with the aluminum oxide powder treated by the roasting at high temperature to prepare the catalyst. The prepared catalyst contains a high content of weak acid and a low content of strong acid, the interaction force between the coagent metal and the pseudo-boehmite powder is weak, which is conducive to the reduction and dispersion of the coagent metal under a low temperature, thereby improving the properties of hydrogenation and removal of oxygen, CO, $CO_2$ and other impurities of the coagent.

**BRIEF DESCRIPTION OF THE DRAWINGS**

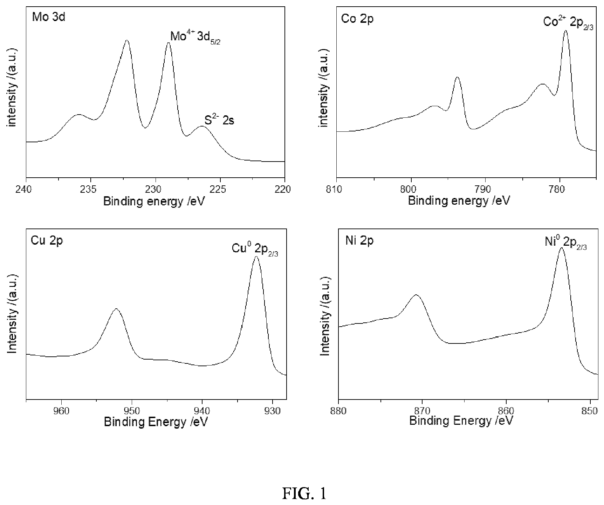

FIG. 1 illustrates an X-ray Photoelectron Spectroscopy (XPS) energy spectrogram of the catalyst in Example 1.

**DESCRIPTION OF THE PREFERRED EMBODIMENT**

The schemes and effects of the invention were further illustrated with reference to Examples, but the embodiments were not intended to limit the invention. In the invention, a commercially available pseudo-boehmite dry gel powder purchased from the Sinopec Catalyst Dalian Branch Company was selected. The pseudo-boehmite dry gel powder had a specific surface area of 392 $m^2$/g, a pore volume of 0.95 $cm^3$/g, a pore diameter of 9.7nm, and a dry basis content of 70 wt%. The valence states of active metals on the catalyst were analyzed and characterized by XPS energy spectrogram. The acid amounts of the catalyst were tested by the $NH_3$-TPD method. The number of lamella layers of the sulfide on the catalyst was obtained by statistics of the TEM photographs. The specific statistical formula was as follows:

$$\overline{N} = \frac{\sum_{i=1}^{n} n_i N_i}{\sum_{i=1}^{n} n_i}$$

Wherein $N$ denoted the average number of the stacked lamella layers; $N_i$ denoted the number of stacked layers of the $i^{th}$ lamella; $n_i$ denoted the statistical number of $N_i$.

Each component of the raw material and the product was obtained by normalized calculation through chromatographic analysis.

Example 1

17.2g of ammonium heptamolybdate and 18.8g of cobalt nitrate hexahydrate were dissolved in a suitable amount of water to prepare 70mL of an active component aqueous solution C. Similarly, 5.8g of copper nitrate and 10.4g of nickel nitrate hexahydrate were dissolved in an appropriate amount of water to prepare 70mL of a coagent component aqueous solution D. A certain amount of the pseudo-boehmite dry gel powder A was then taken to perform roasting at 1,000°C in an air atmosphere for 1.5h, and the aluminum oxide dry gel powder B was obtained. 100g of the roasted aluminum oxide dry gel powder B was impregnated in the loading aqueous solution C, then subjected to drying at 100°C for 3h, subsequently subjected to roasting at 1,000°C in an air atmosphere for 1.5h, and further treated at 350°C in the mixed atmosphere of $H_2S$ and $H_2$ with the $H_2S$ content of 1% for 6h, the powder E was obtained. 100g of the pseudo-boehmite dry gel powder A was impregnated in a loading aqueous solution D, the impregnated pseudo-boehmite dry gel powder A was subjected to drying at 100°C for 3h, then roasting at 650°C in a hydrogen gas atmosphere for 4h, the powder F was obtained. Powder E and powder F were uniformly mixed, and the mixture was crushed and sieved (200 meshes); 2g of sesbania powder, 15g of 10% nitric acid, and 150mL of deionized water were further added, subjected to molding, and drying at 100°C for 3h, and roasting at 350°C in a nitrogen atmosphere for 2h, the final catalyst C-1 was prepared. According to NIST (National Institute of Standards and Technology in the United States of America) XPS database queries, the metal Mo was +4

tetravalent cation in MoS$_2$, Co was +2 divalent cation in CoS, both Cu and Ni were 0 valence metal states, as shown in FIG. 1.

Example 2

29.0g of ammonium heptamolybdate and 26.0g of nickel nitrate hexahydrate were dissolved in a suitable amount of water to prepare 90mL of an active component aqueous solution C. Similarly, 3.2g of silver nitrate and 6.3g of ammonium metatungstate were dissolved in an appropriate amount of water to prepare 42mL of a coagent component aqueous solution D. A certain amount of the pseudo-boehmite dry gel powder A was then taken to perform roasting at 900°C in an air atmosphere for 3h, and the alumina oxide dry gel powder B was obtained. 120g of the roasted aluminum oxide dry gel powder B was impregnated in the loading aqueous solution C, then subjected to drying at 110°C for 3h, subsequently subjected to roasting at 1,050°C in an air atmosphere for 1h, and further treated at 400°C in the mixed atmosphere of H$_2$S and H$_2$ with the H$_2$S content of 1% for 5h, the powder E was obtained. 60g of pseudo-boehmite dry gel powder A was impregnated in a loading aqueous solution D, the impregnated pseudo-boehmite dry gel powder A was subjected to drying at 110°C for 3h, then roasting at 550°C in a hydrogen gas atmosphere for 3h, the powder F was obtained. The powder E and powder F were uniformly mixed, and the mixture was crushed and sieved (200 meshes); 2.5g of sesbania powder, 17g of 10% nitric acid, and 190mL of deionized water were further added, subjected to molding, and drying at 110°C for 3h, and roasting at 250°C in a nitrogen atmosphere for 2h, the final catalyst C-2 was prepared.

Example 3

19.1g of ammonium metatungstate, 7.3g of ammonium heptamolybdate, and 10.6g of nickel nitrate hexahydrate were dissolved in a suitable amount of water to prepare 80mL of an active component aqueous solution C. Similarly, 1.6g of cobalt nitrate and 2.5g of nickel nitrate were dissolved in an appropriate amount of water to prepare 35mL of a coagent component aqueous solution D. A certain amount of the pseudo-boehmite dry gel powder A was then taken to perform roasting at 800°C in an air atmosphere for 5h, and the aluminum oxide dry gel powder B was obtained. 100g of the roasted aluminum oxide dry gel powder B was impregnated in the loading aqueous solution C, then subjected to drying at 110°C for 2h, subsequently subjected to roasting at 900°C in an air atmosphere for 4h, and further treated at 500°C in the mixed atmosphere of H$_2$S and H$_2$ with the H$_2$S content of 1% for 4h, the powder E was obtained. 50g of the pseudo-boehmite dry gel powder A was impregnated in a loading aqueous solution D, the impregnated pseudo-boehmite dry gel powder A was subjected to drying at 110°C for 2h, then roasting at 450°C in a hydrogen gas atmosphere for 2h, the powder F was obtained. The powder E and powder F were uniformly mixed, and the mixture was crushed and sieved (200 meshes); 2.5g of sesbania powder, 17g of 10% nitric acid, and 170mL of deionized water were further added, subjected to molding, and drying at 110°C for 2h, and roasting at 300°C in a nitrogen atmosphere for 2h, the final catalyst C-3 was prepared.

Example 4

24.4g of ammonium metatungstate, 9.3g of ammonium heptamolybdate, and 13.5g of nickel nitrate hexahydrate were dissolved in a suitable amount of water to prepare 80mL of active component aqueous solution C. Similarly, 9.3g of copper nitrate was dissolved in an appropriate amount of water to prepare 70mL of a coagent component aqueous solution D. A certain amount of the pseudo-boehmite dry gel powder A was then taken to perform roasting at 900°C in an air atmosphere for 5h, and the aluminum oxide dry gel powder B was obtained. 100g of the roasted aluminum oxide dry gel powder B was impregnated in the loading aqueous solution C, then subjected to drying at 90°C for 4h, subsequently subjected to roasting at 850°C in an air atmosphere for 4h, and further treated at 300°C in the mixed atmosphere of H$_2$S and H$_2$ with the H$_2$S content of 1% for 7h, the powder E was obtained. 100g of the pseudoboehmite dry gel powder A was impregnated in a loading aqueous solution D, the impregnated pseudo-boehmite dry gel powder A was subjected to drying at 90°C for 4h, then roasting at 600°C in a hydrogen gas atmosphere for 3h, the powder F was obtained. The powder E and powder F were uniformly mixed, and the mixture was crushed and sieved (200 meshes); 2.5g of sesbania powder, 20g of 10% nitric acid, and 210mL of deionized water were further added, subjected to molding, and drying at 90°C for 4h, and roasting at 300°C in a nitrogen atmosphere for 2h, the final catalyst C-4 was prepared.

Example 5

A certain amount of the pseudo-boehmite dry gel powder A was then taken to perform roasting at 950°C in an air atmosphere for 1.5h, the aluminum oxide dry gel powder B was obtained. 12.8g of ammonium heptamolybdate and 13.0g of cobalt nitrate hexahydrate were then taken and dissolved in an appropriate amount of water to prepare 100mL of an active component aqueous solution C. 100g of the roasted aluminum oxide dry gel powder B impregnated in the loading aqueous solution C, then subjected to drying at 100°C for 3h, subsequently subjected to roasting at 1,050°C in an air atmosphere for 1.5h, and further treated at 400°C in the mixed atmosphere of $H_2S$ and $H_2$ with the $H_2S$ content of 1% for 6h, the powder D was obtained. Powder D was crushed and sieved (200 meshes); 2.5g of sesbania powder, 20g of 10% nitric acid, and 100mL of deionized water were further added, subjected to molding, and drying at 100°C for 3h, and roasting at 350°C in a nitrogen atmosphere for 2h, the final catalyst C-5 was prepared.

Comparative Example 1

The catalyst was prepared according to the method in Example 1, except that the pseudo-boehmite powder was not subjected to roasting at high temperature during the preparation process. 13.7g of ammonium heptamolybdate and 14.9g of cobalt nitrate hexahydrate were dissolved in a suitable amount of water to prepare 100mL of an active component aqueous solution C. Similarly, 4.6g of copper nitrate and 8.2g of nickel nitrate hexahydrate were dissolved in an appropriate amount of water to prepare 70mL of a coagent component aqueous solution D. 142.9g of the pseudo-boehmite dry gel powder A was then taken to be impregnated in the loading solution C, then subjected to drying at 100°C for 3h, subsequently subjected to roasting at 1,000°C in an air atmosphere for 1.5h, and further treated at 350°C in the mixed atmosphere of $H_2S$ and $H_2$ with the $H_2S$ content of 1% for 6h, the powder E was obtained. 100g of the pseudo-boehmite dry gel powder A was impregnated in a loading aqueous solution D, the impregnated pseudo-boehmite dry gel powder A was subjected to drying at 100°C for 3h, then roasting at 650°C in a hydrogen gas atmosphere for 4h, the powder F was obtained. Powder E and powder F were uniformly mixed, and the mixture was crushed and sieved (200 meshes); 2g of sesbania powder, 15g of 10% nitric acid, and 150mL of deionized water were further added, subjected to molding, and drying at 100°C for 3h, and roasting at 350°C in a nitrogen atmosphere for 2h, the final catalyst D-1 was prepared.

Comparative Example 2

The catalyst was prepared according to the method in Example 1, except that the roasting at high temperature in step (1) was canceled, and the pseudo-boehmite powder was subjected to roasting at low temperature to obtain γ-aluminum oxide. 13.7g of ammonium heptamolybdate and 14.9g of cobalt nitrate hexahydrate were taken and dissolved in a suitable amount of water to prepare 100mL of an active component aqueous solution C. Similarly, 4.6g of copper nitrate and 8.2g of nickel nitrate hexahydrate were dissolved in an appropriate amount of water to prepare 70mL of a coagent component aqueous solution D. A certain amount of pseudo-boehmite dry gel powder A was then taken to perform roasting at 550°C in an air atmosphere for 3h to obtain γ-aluminum oxide dry gel powder, 100g of γ-aluminum oxide dry gel powder was then taken to be impregnated in the loading solution C, subjected to drying at 100°C for 3h, subsequently subjected to roasting at 1,000°C in an air atmosphere for 1.5h, and further treated at 350°C in the mixed atmosphere of $H_2S$ and $H_2$ with the $H_2S$ content of 1% for 6h, the powder E was obtained. 100g of the pseudo-boehmite dry gel powder A was impregnated in a loading aqueous solution D, the impregnated pseudo-boehmite dry gel powder A was subjected to drying at 100°C for 3h, then roasting at 650°C in a hydrogen gas atmosphere for 4h, the powder F was obtained. Powder E and powder F were uniformly mixed, and the mixture was crushed and sieved (200 meshes); 2g of sesbania powder, 15g of 10% nitric acid, and 150mL of deionized water were further added, subjected to molding, and drying at 100°C for 3h, and roasting at 350°C in a nitrogen atmosphere for 2h, the final catalyst D-2 was prepared.

Comparative Example 3

The catalyst was prepared according to the method in Example 1, except that the powder impregnated with Group VIB and/or Group VIII metals was not subjected to roasting at high temperature during the preparation process. 13.7g of ammonium heptamolybdate and 14.9g of cobalt nitrate hexahydrate were dissolved in a suitable amount of water to prepare 100mL of an active component aqueous solution C. Similarly, 4.6g of copper nitrate and 8.2g of nickel nitrate hexahydrate were dissolved in an appropriate amount of water to prepare 70mL of a coagent component aqueous solution D. A certain amount of the pseudo-boehmite dry gel powder A was taken and subjected to roasting at 1,000°C in an air atmosphere for 1.5h to obtain an alumina oxide dry gel powder B. 100g of aluminum oxide dry gel powder B was then taken to be impregnated in the loading solution C, then subjected to drying at 100°C for 3h, and further treated at 350°C in the mixed atmosphere of $H_2S$ and $H_2$ with the $H_2S$ content of 1% for 6h, the powder E was obtained. 100g of the pseudo-boehmite dry gel powder A was impregnated in a loading aqueous solution D, the impregnated pseudo-boehmite dry gel powder A was subjected to drying at 100°C for 3h, then roasting at 650°C in a hydrogen gas atmosphere for 4h, the powder F was obtained. Powder E and powder F were uniformly mixed, and the mixture was crushed and sieved (200 meshes); 2g of sesbania powder, 15g of 10% nitric acid, and 150mL of deionized water were further added, subjected to molding, and drying at 100°C for 3h, and roasting at 350°C in a nitrogen atmosphere for 2h, the final catalyst D-3 was prepared.

Comparative Example 4

The catalyst was prepared according to the method in Example 1, except that the carrier was initially prepared and then impregnated with the active metal component and a coagent component in steps. A certain amount of the pseudo-boehmite dry gel powder A was taken to perform roasting at 1,000°C in an air atmosphere for 1.5h, to obtain an alumina oxide dry gel powder B. 100g of roasted aluminum oxide dry gel powder B and 100g of pseudo-boehmite dry gel powder A were subsequently mixed uniformly, crushed and sieved (200 meshes), 2g of sesbania powder, 15g of 10% nitric acid, and 150mL of deionized water were further added, subjected to molding, and drying at 100°C for 3h, and roasting at 250°C in an air atmosphere for 2h, the catalyst carrier was prepared. 13.7g of ammonium heptamolybdate and 14.9g of cobalt nitrate hexahydrate were dissolved in a suitable amount of water to prepare 70mL of an active component aqueous solution C. Similarly, 4.6g of copper nitrate and 8.2g of nickel nitrate hexahydrate were dissolved in an appropriate amount of water to prepare 35mL of a coagent component aqueous solution D. The prepared carrier was impregnated in the loading solution C, then subjected to drying at 100°C for 3h, and subjected to roasting at 1,000°C in an air atmosphere for 1.5h, and further treated at 350°C in the mixed atmosphere of $H_2S$ and $H_2$ with the $H_2S$ content of 1% for 6h, the first-stage catalyst E was obtained. The first-stage catalyst E was impregnated in a loading aqueous solution D, subjected to drying at 100°C for 3h, then roasting at 350°C in a nitrogen atmosphere for 2h, and the final catalyst D-4 was prepared.

Comparative Example 5

12.8g of ammonium heptamolybdate and 13.0g of cobalt nitrate hexahydrate were dissolved in a suitable amount of water to prepare 100mL of an active component aqueous solution C. 142.9g of the pseudo-boehmite dry gel powder A was then impregnated in the loading solution C, subjected to drying at 100°C for 3h and roasting at 450°C in an air atmosphere for 1.5h, and further treated at 400°C in the mixed atmosphere of $H_2S$ and $H_2$ with the $H_2S$ content of 1% for 6h, the powder D was obtained. Powder D was crushed and sieved (200 meshes); 2.5g of sesbania powder, 20g of 10% nitric acid, and 100mL of deionized water were further added, subjected to molding, and drying at 100°C for 3h, and roasting at 350°C in a nitrogen atmosphere for 2h, the final catalyst D-5 was prepared.

The component contents and properties of said catalysts were listed in Table 1.

Table 1 Component contents and properties of the catalysts in Examples and Comparative Examples

| | C-1 | C-2 | C-3 | C-4 | C-5 | D-1 | D-2 | D-3 | D-4 | D-5 |
|---|---|---|---|---|---|---|---|---|---|---|
| Group VIB sulfide, wt% | 8.0 | 13.0 | 16.0 | 16.0 | 10.0 | 8.0 | 8.0 | 8.0 | 8.0 | 10.0 |
| Group VIII sulfide, wt% | 3.0 | 4.0 | 2.0 | 2.0 | 3.5 | 3.0 | 3.0 | 3.0 | 3.0 | 3.5 |
| Coagent c, wt% | 2.0 | 3.0 | 0.5 | 1.5 | 0 | 2.0 | 2.0 | 2.0 | 2.0 | 0 |
| Total acid amount, mmol/g | 0.38 | 0.25 | 0.15 | 0.32 | 0.17 | 0.47 | 0.45 | 0.35 | 0.08 | 0.67 |
| Strong acid amount at 400-500°C, % | 6.8 | 9.4 | 13.2 | 13.6 | 3.5 | 19.8 | 17.4 | 10.5 | 4.2 | 22.6 |
| Moderate strength acid amount at 250-400°C, % | 17.6 | 12.2 | 15.5 | 14.1 | 14.6 | 27.5 | 35.7 | 19.4 | 10.4 | 47.9 |
| Average number of lamella layers of sulfide | 1.2 | 1.3 | 1.3 | 1.3 | 1.3 | 1.4 | 1.6 | 1.8 | 1.6 | 2.7 |
| Proportion of a single layer in sulfide, % | 78 | 72 | 70 | 68 | 73 | 60 | 47 | 40 | 50 | 32 |

Coking dry gas hydrogenation

The catalysts were subjected to the activity evaluation test in a 10mL reaction device, the reaction pressure was 3.0MPa, the reaction temperature was 190°C, the gas hourly space velocity was 700h$^{-1}$, the raw material properties were shown in Table 2, the evaluation results after reaction for 500h were shown in Table 3, the carbon deposition amounts on the catalysts were shown in Table 4.

Table 2 Feedstock composition of the coking dry gas

| Composition | Content, mol% |
|---|---|
| $H_2$ | 9.135 |

(continued)

| Composition | Content, mol% |
|---|---|
| $O_2$ | 0.092 |
| $N_2$ | 0.026 |
| CO | 0.238 |
| $CO_2$ | 0.516 |
| $CH_4$ | 13.762 |
| Alkane (C2-C5) | 70.483 |
| Olefins (C2-C4) | 5.596 |
| $H_2S$ | 0.099 |
| Carbonyl sulfide | 0.038 |
| $H_2O$ | 0.015 |
| Total | 100 |

Table 3 Composition of the hydrogenation product after reaction for 500h

| Product compositio n, mol% | C-1 | C-2 | C-3 | C-4 | D-1 | D-2 | D- | D-4 | D-5 |
|---|---|---|---|---|---|---|---|---|---|
| $H_2$ | 2.823 | 2.383 | 2.408 | 2.854 | 5.403 | 6.186 | 5.623 | 4.415 | 6.334 |
| $O_2$ | 0 | 0 | 0 | 0 | 0.002 | 0.002 | 0.002 | 0.004 | 0.009 |
| $N_2$ | 0.027 | 0.026 | 0.027 | 0.027 | 0.026 | 0.026 | 0.026 | 0.026 | 0.026 |
| CO | 0 | 0 | 0 | 0 | 0.005 | 0.006 | 0.005 | 0.038 | 0.042 |
| $CO_2$ | 0 | 0 | 0.001 | 0 | 0.006 | 0.006 | 0.005 | 0.055 | 0.057 |
| $CH_4$ | 15.29 4 | 15.59 4 | 16.14 6 | 15.88 8 | 14.66 7 | 14.04 7 | 13.84 3 | 14.65 9 | 14.68 |
| Alkane (C2-C5) | 79.53 2 | 79.81 1 | 79.25 1 | 79.06 6 | 77.57 1 | 77.13 | 78.00 3 | 78.46 5 | 76.55 7 |
| Olefins (C2-C4) | 0.699 | 0.636 | 0.633 | 0.63 | 1.022 | 1.332 | 1.128 | 1.033 | 1.121 |
| $H_2S$ | 0.14 | 0.144 | 0.132 | 0.132 | 0.12 | 0.125 | 0.11 | 0.126 | 0.142 |
| Organic sulfur | 0 | 0 | 0 | 0 | 0.005 | 0.007 | 0.01 | 0.002 | 0.004 |
| $H_2O$ | 1.485 | 1.406 | 1.402 | 1.403 | 1.173 | 1.133 | 1.245 | 1.177 | 1.028 |
| Total | 100 | 100 | 100 | 100 | 100 | 100 | 100 | 100 | 100 |

Table 4 Carbon deposition amounts of catalysts after hydrogenation

|  | C-1 | C-2 | C-3 | C-4 | D-1 | D-2 | D-3 | D-4 | D-5 |
|---|---|---|---|---|---|---|---|---|---|
| Carbon deposition amount, wt% | 2.07 | 2.04 | 1.93 | 2.29 | 3.24 | 3.43 | 2.99 | 2.96 | 4.62 |

The evaluation results in Table 3 show that the activity of olefin saturation and the activity of removing impurities (e.g., sulfur, oxygen, CO, $CO_2$) of the catalysts in Examples of the invention are better than those of catalysts in Comparative Examples, demonstrate that the catalysts in the invention are more suitable for hydrogenation reaction of the coking dry gas. The carbon deposition amounts of the catalysts after hydrogenation in Table 4 shows that the carbon deposition amounts of the catalysts in Examples of the invention are obviously lower than those of the catalysts in Comparative Examples after reaction for 500 hours, it indicates that the catalysts in Examples of the invention have better stability and are more suitable for long-term industrial operation.

Liquefied gas hydrogenation

The catalysts were subjected to an activity evaluation test in a 10mL reaction device, the reaction pressure was 1.5MPa, the reaction temperature was 180°C, the liquid hourly space velocity was $4.0h^{-1}$, and the volume ratio of hydrogen/oil was 200: 1. The raw material was coking liquefied gas with a total olefin content of 20 v% and a total sulfur content of 203 $mg/m^3$. The evaluation results after the reaction for 500 hours were shown in Table 5, and the carbon deposition amounts of the catalysts after hydrogenation were shown in Table 6.

Table 5 Composition of the product obtained after reaction for 500h

| | A total olefin content of the product, v% | Total sulfur content of product, $mg/m^3$ |
|---|---|---|
| C-1 | 0.003 | 0.8 |
| C-2 | 0.005 | 1.0 |
| C-3 | 0.004 | 0.7 |
| C-4 | 0.006 | 0.9 |
| C-5 | 0.006 | 1.0 |
| D-1 | 0.8 | 3.9 |
| D-2 | 1.1 | 4.1 |
| D-3 | 0.7 | 3.4 |
| D-4 | 1.0 | 3.6 |
| D-5 | 1.4 | 4.9 |

Table 6 Carbon deposition amounts of catalysts after hydrogenation

| | C-1 | C-2 | C-3 | C-4 | C-5 | D-1 | D-2 | D-3 | D-4 | D-5 |
|---|---|---|---|---|---|---|---|---|---|---|
| Carbon deposition amounts, wt% | 2.44 | 2.12 | 2.38 | 2.23 | 2.41 | 3.84 | 3.92 | 3.66 | 3.73 | 4.34 |

The evaluation results in Table 5 and the carbon deposit amount of the catalysts after the hydrogenation in Table 6 demonstrate that the olefin saturation and the desulfurization activity of the catalysts in Examples of the invention are better than those of the catalysts in Comparative Example catalyst, and the carbon deposit amounts of the catalysts in Examples after reaction for 500 hours are lower, it shows that the catalysts in Examples have better stability.

The above content describes in detail the preferred embodiments of the present invention, but the present invention is not limited thereto. A variety of simple modifications can be made in regard to the technical solutions of the present invention within the scope of the technical concept of the present invention, including a combination of individual technical features in any other suitable manner, such simple modifications and combinations thereof shall also be regarded as the content disclosed by the present invention, each of them falls into the protection scope of the present invention.

**Claims**

1. A hydrogenation catalyst, is **characterized in that** the hydrogenation catalyst comprises a carrier component and an active hydrogenation component; the carrier component comprises aluminum oxide; and the active hydrogenation component is present in the form of a sulfide on the catalyst, the average number of lamella layers of the sulfide is less than 1.5, and the proportion of a single layer is within the range of 65%-80%.

2. The catalyst according to claim 1, wherein the average number of lamella layers of the sulfide is within the range of 1.2-1.3; and/or

   the hydrogenation catalyst comprises a coagent including one or more of Cu, Ag, Ni, Co, W, and Mo;
   preferably, the coagent is present in the form of a metallic state on the catalyst;
   preferably, the coagent content in the catalyst is from 0.1wt% to 4wt%, more preferably from 0.3wt% to 4wt%, further preferably from 0.5wt% to 3wt%.

3. The catalyst according to claim 1 or 2, wherein the content of active hydrogenation component calculated in terms of sulfide in the total mass of the catalyst is from 1.5wt% to 30wt%, preferably from 10wt% to 20wt%, based on the weight of said catalyst.

4. The catalyst according to any one of claims 1-3, wherein the active hydrogenation component comprises Group VIB metals and Group VIII metals;

   preferably, Group VIB metals comprise Mo and/or W; Group VIII metals comprise Co and/or Ni;
   preferably, the content of Group VIB metal sulfide in the total mass of the catalyst is from 1wt% to 20wt%, more preferably from 8wt% to 16wt%, the content of Group VIII metal sulfide in the total mass of the catalyst is from 0.5wt% to 10wt%, more preferably from 2wt% to 4wt%, based on the weight of the catalyst.

5. The catalyst according to any one of claims 1-4, wherein the total acid amount of the catalyst is within the range of 0.1-0.4 mmol/g, wherein the content of strong acid amount at 400-500°C is within the range of 5-15%, the content of moderate strength acid amount at 250-400°C is within the range of 10-20%, and the balance is weak acid at 150-250°C.

6. A preparation method of a hydrogenation catalyst, is **characterized in that** the preparation method comprises the following steps:

   (I) taking aluminum oxide powder A and roasting same at high temperature in an oxygen-containing atmosphere to obtain aluminum oxide dry gel powder B;
   (II) impregnating the aluminum oxide dry gel powder B by using the active hydrogenation component impregnating solution C, drying, roasting at high temperature in the oxygen-containing atmosphere, and sulfidizing same to obtain powder E;
   (III) molding, drying, and roasting the powder E prepared in step (II);

   wherein the respective temperature of high-temperature roasting in step (I) and step (II) are 800°C or above.

7. The preparation method according to claim 6, wherein the method further comprises the following steps: impregnating the aluminum oxide powder A with a coagent impregnating solution D, drying the impregnated aluminum oxide powder A and reducing to obtain powder F;

   step (III) comprises: mixing powder E with powder F, and then molding, drying, and roasting the mixture;
   preferably, the reduction treatment includes roasting in a reducing atmosphere, more preferably roasting in a hydrogen atmosphere;
   the roasting conditions in the reduction treatment comprise a roasting temperature within the range of 300-700°C and a roasting time within the range of 2-5 hours; preferably, the drying conditions before reduction comprise a drying time within the range of 1-5 hours and a drying temperature within the range of 80-120°C; preferably, the impregnation method of the coagent impregnation is saturated impregnation or supersaturated impregnation.

8. The method according to claim 6 or 7, wherein physicochemical parameters of the aluminum oxide powder A comprise: a specific surface area within the range of 100-480 $m^2$/g, a pore volume within the range of 0.4-1.2 $cm^3$/g, and a pore diameter within the range of 4.0-35.0nm.

9. The method according to any one of claims 6-8, wherein in step (I),

   the oxygen-containing atmosphere has an oxygen content from 10 v% to 30 v%, preferably an air atmosphere; and/or
   the roasting conditions at high temperatures comprise a roasting temperature within the range of 800-1,100°C and a roasting time within the range of 0.5-5 hours.

10. The method according to any one of claims 6-9, wherein in step (II),

    the oxygen-containing atmosphere has an oxygen content from 10 v% to 30 v%, preferably an air atmosphere; and/or
    the roasting conditions at high temperatures comprise a roasting temperature within the range of 800-1,100°C and a roasting time within the range of 0.5-5 hours.

11. The method according to any one of claims 6-10, wherein the sulfidizing in step (II) comprises: roasting in a vulcanization atmosphere, preferably roasting in a mixed atmosphere of $H_2S$ and $H_2$, and more preferably, the content of $H_2S$ in the mixed atmosphere of $H_2S$ and $H_2$ is within the range of 0.1 v%-2 v%;
preferably, the roasting conditions comprise a roasting temperature within the range of 250-550°C and a roasting time within the range of 3-8 hours.

12. The method according to any one of claims 6-11, wherein the impregnation method of step (II) is saturated impregnation or supersaturated impregnation.

13. The method according to any one of claims 6-12, wherein the catalyst molding process in step (III) comprises: adding an extrusion aid, a peptizing agent, and water into the composite powder and blending to form a plastic body, then kneading and molding the plastic body;

preferably, the extrusion aid is one or more selected from the group consisting of methylcellulose, sesbania powder, starch, and polyvinyl alcohol;
preferably, the peptizing agent is one or more selected from the group consisting of dilute nitric acid, dilute phosphoric acid, and silicic acid.

14. The method according to any one of claims 6-13, wherein the drying conditions in steps (II) and (III) respectively comprise a drying time within the range of 1-5 hours and a drying temperature within the range of 80-120°C.

15. The method according to any one of claims 6-14, wherein the roasting condition in step (III) comprises a roasting temperature within the range of 200-350°C and a roasting time within the range of 1-4 hours; and/or
the roasting atmosphere is an inert atmosphere, which is one or more gases selected from $N_2$, He, or Ar.

16. Use of the hydrogenation catalyst according to any one of claims 1-5 in the hydrogenation of ethylene cracking raffinate oil, liquefied gas, or dry gas, preferably the use in the reactions for producing low carbon hydrocarbons from dry gas hydrogenation,
preferably, the reaction conditions for the reaction for producing low carbon hydrocarbon from dry gas hydrogenation comprise a reaction pressure within the range of 0.1-10MPa, a gas hourly space velocity within the range of 300-10,000 $h^{-1}$, and a reaction temperature within the range of 150-400°C.

FIG. 1

## INTERNATIONAL SEARCH REPORT

| International application No. |
| --- |
| **PCT/CN2023/136371** |

**A. CLASSIFICATION OF SUBJECT MATTER**

B01J27/051(2006.01)i; B01J27/049(2006.01)i; B01J27/047(2006.01)i; C10G45/08(2006.01)i

According to International Patent Classification (IPC) or to both national classification and IPC

**B. FIELDS SEARCHED**

Minimum documentation searched (classification system followed by classification symbols)

IPC:B01J27; C10G45

Documentation searched other than minimum documentation to the extent that such documents are included in the fields searched

Electronic data base consulted during the international search (name of data base and, where practicable, search terms used)

CNTXT, ENTXTC, WPABSC, WPABS, DWPI, CJFD, 中国期刊网全文数据库, China Journal Network Full-text Database (CJFD): 孙进, 郭蓉, 李扬, 牛世坤, 段为宇, 加氢, 催化剂, 硫化, 单层, 焙烧, hydrogenation, catalyst, vulcanization, sulfuration, monolayer, single layer, calcinate, roast

**C. DOCUMENTS CONSIDERED TO BE RELEVANT**

| Category* | Citation of document, with indication, where appropriate, of the relevant passages | Relevant to claim No. |
| --- | --- | --- |
| X | US 4853359 A (SIMON FRASER UNIVERSITY) 01 August 1989 (1989-08-01) description, column 2 paragraphs 1-3, column 4 paragraph 1, and column 5 paragraph 4 | 1, 3-5, 16 |
| Y | US 4853359 A (SIMON FRASER UNIVERSITY) 01 August 1989 (1989-08-01) description, column 2 paragraphs 1-3, column 4 paragraph 1, and column 5 paragraph 4 | 2-5, 16 |
| Y | CN 112705227 A (CHINA PETROLEUM & CHEMICAL CORP. et al.) 27 April 2021 (2021-04-27) description, paragraphs 7 and 16 | 2-5, 16 |
| X | US 4396500 A (UNION OIL COMPANY OF CALIFORNIA) 02 August 1983 (1983-08-02) description, column 4 paragraph 3-column 5 paragraph 2, and column 2 paragraph 2 | 6, 8-15 |
| A | CN 112973737 A (CHINA PETROLEUM & CHEMICAL CORP. et al.) 18 June 2021 (2021-06-18) entire document | 1-16 |
| A | CN 111196935 A (CHINA NATIONAL PETROLEUM CORP.) 26 May 2020 (2020-05-26) entire document | 1-16 |

☑ Further documents are listed in the continuation of Box C. ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
| --- | --- |
| **19 February 2024** | **29 February 2024** |

| Name and mailing address of the ISA/CN | Authorized officer |
| --- | --- |
| **China National Intellectual Property Administration (ISA/ CN) China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

<table>
<tr><td>International application No.</td></tr>
<tr><td>**PCT/CN2023/136371**</td></tr>
</table>

**C.    DOCUMENTS CONSIDERED TO BE RELEVANT**

| Category* | Citation of document, with indication, where appropriate, of the relevant passages | Relevant to claim No. |
|---|---|---|
| A | CN 107961771 A (CHINA PETROLEUM & CHEMICAL CORP. et al.) 27 April 2018 (2018-04-27)<br>entire document | 1-16 |

Form PCT/ISA/210 (second sheet) (July 2022)

**INTERNATIONAL SEARCH REPORT**
Information on patent family members

International application No.

**PCT/CN2023/136371**

| Patent document cited in search report | | | Publication date (day/month/year) | Patent family member(s) | | | Publication date (day/month/year) |
|---|---|---|---|---|---|---|---|
| US | 4853359 | A | 01 August 1989 | EP | 0242804 | A2 | 28 October 1987 |
| | | | | EP | 0242804 | A3 | 13 July 1988 |
| | | | | CA | 1302383 | C | 02 June 1992 |
| | | | | EP | 242804 | A | 28 October 1987 |
| CN | 112705227 | A | 27 April 2021 | CN | 112705227 | B | 11 November 2022 |
| US | 4396500 | A | 02 August 1983 | | None | | |
| CN | 112973737 | A | 18 June 2021 | CN | 112973737 | B | 05 April 2022 |
| CN | 111196935 | A | 26 May 2020 | CN | 111196935 | B | 01 February 2022 |
| CN | 107961771 | A | 27 April 2018 | CN | 107961771 | B | 13 November 2020 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202211569861
- CN 101722006 A
- CN 200910230393
- CN 112742399 A
- CN 201911052623
- CN 112742408 A
- CN 201911053316